(19) Europäisches Patentamt
European Patent Office
Office européen des brevets

(11) Publication number: 0 000 815
B1

(12) **EUROPEAN PATENT SPECIFICATION**

(45) Date of publication of patent specification: 27.01.82

(51) Int. Cl.$^3$: C 07 C 125/06

(21) Application number: 78300188.6

(22) Date of filing: 24.07.78

# (54) Process for preparing N-aryl or N-aralkyl substituted urethanes.

(30) Priority: 25.07.77 JP 88400/77

(43) Date of publication of application:
21.02.79 Bulletin 79/4

(45) Publication of the grant of the European patent:
27.01.82 Bulletin 82/4

(84) Designated Contracting States:
DE FR GB NL

(56) References cited:
NL - C - 94 613

CHEMICAL ABSTRACTS, vol. 77 (1972) 87801y.

(73) Proprietor: MITSUI TOATSU CHEMICALS, INCORPORATED
2-5 3-chome Kasumigaseki
Chiyoda-Ku Tokyo 100 (JP)

(72) Inventor: Miyata, Katsuharu
300, Hirabaru-machi
Omuta-shi Fukuoka-ken (JP)
Inventor: Aiga, Makoto
300, Hirabaru-machi
Omuta-shi Fukuoka-ken (JP)
Inventor: Hasegawa, Seiji
50, Katahira-machi
Omuta-shi Fukuoka-ken (JP)

(74) Representative: Ellis, John Clifford Holgate et al,
MEWBURN ELLIS & CO. 70-72 Chancery Lane
London WC2A 1AD (GB)

EP 0 000 815 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Process for preparing n-aryl or N-aralkyl substituted urethanes

This invention relates to a process for preparing N-aryl or N-aralkyl substituted urethanes. More particularly, it relates to a process for preparing N-aryl or N-aralkyl substituted urethanes by reacting (i) an aryl or aralkyl nitrogen-containing compound, (ii) an organic compound containing at least one hydroxyl group, and (iii) carbon monoxide in the presence of a catalyst.

There have recently been proposed a large number of processes for producing N-aryl or N-aralkyl substituted urethanes by reacting aryl or aralkyl nitro compounds, organic compounds containing at least one hydroxyl group (hereinafter referred to simply as hydroxyl group-containing compound), and carbon monoxide in the presence of catalysts.

For example, U.S. Patent Specification No. 3,338,956 describes a process using rhodium chlorocarbonyl as a catalyst for the urethanation reaction. Further, British Patent Specification No. 1,543,051 discloses a process in which the urethanation reaction is conducted with rhodium chlorocarbonyl as the catalyst and a multi-valent metal halide as the promoter. In these processes, however, aryl or aralkyl nitro compounds are essentially used as the aryl or aralkyl nitrogen-containing compounds to permit the nitro group or groups to take part in the urethanation reaction.

As a result of an extensive study of a process of producing N-aryl and N-aralkyl urethanes, we have found an improved process for producing N-aryl and N-aralkyl substituted urethanes in which the urethanes are produced from specified aromatic aryl and aralkyl primary amine compounds, carbon monoxide and hydroxyl group-containing compounds.

According to the present invention there is provided a process for producing N-aryl or N-aralkyl substituted urethanes by reacting (i) an aryl or aralkyl nitrogen-containing compound, (ii) an organic compound having at least one hydroxyl group, and (iii) carbon monoxide in the presence of a catalyst, characterised in that component (i) is an aryl or aralkyl primary amino compound having a nitro group, a nitroso group or a carbamate group, that the catalyst consists of a catalytic system comprising a metal and/or a compound thereof chosen from palladium, rhodium and ruthenium and compounds thereof and as a promoter a Lewis acid and/or a compound thereof, and that the reaction is conducted at elevated temperature and under a carbon monoxide partial pressure of 10—1000kg/cm$^2$. Preferably component (i) is an aryl primary amino compound and preferably component (i) has a nitro group.

It is known that if amino compounds having a nitro group as a substituent are subjected to a urethanation reaction in the presence of the catalytic system indicated above, the nitro group can be converted into a carbamate. However, it cannot be expected at all from prior art processes that the amino group is also convertible into a carbamate. It has also been found that where aryl and aralkyl amino compounds which are free of a nitro group, a nitroso group or a carbamate group are used as the starting material, no urethanation reaction takes place. This is a hitherto unknown and rather amazing fact concerning the production of N-aryl or N-aralkyl substituted urethanes from aryl or aralkyl primary amino compounds, carbon monoxide and hydroxyl group-containing compounds.

As mentioned hereinabove, the aryl and aralkyl amino compounds which are employed as the chief starting material are aryl and aralkyl primary amino compounds having a nitro group, a nitroso group or a carbamate group. Examples of such aryl and aralkyl compounds include *m*- and *p*-nitroaniline, *m*- and *p*-nitrosoaniline, aniline *m*- and *p*-carbamate, 2-amino-4-nitrotoluene, 2-amino-4-nitrosotoluene, 2-aminotoluene-4-carbamate, 2-nitro-4-aminotoluene, 2-nitroso-4-aminotoluene, 4-aminotoluene-2-carbamate, 2-amino-6-nitrotoluene, 2-amino-6-nitrosotoluene, 2-aminotoluene-6-carbamate, 4-amino-4′-nitrobiphenyl, 4-amino-4′-nitrosobiphenyl 4-aminobiphenyl-4′-carbamate, 2-amino-4-nitrobiphenyl, 2-amino-4-nitrosobiphenyl, 2-aminobiphenyl-4-carbamate, 2-nitro-4-aminobiphenyl, 2-nitroso-4-aminobiphenyl, 4-aminobiphenyl-2-carbamate, 4-amino-4′-nitrodibenzyl, 4-amino-4′-nitrosodibenzyl, 4-aminodibenzyl-4′-carbamate, 4-amino-4′-nitrodiphenylmethane, 4-amino-4′-nitrosodiphenylmethane, 4-aminodiphenylmethane-4′-carbamate, 4-amino-4′-nitrodiphenyl ether, 4-amino-4′-nitrosodiphenyl ether, 4-aminodiphenyl ether 4′-carbamate, bis(2-amino-4-nitrophenyl)-ether, bis(2-amino-4-nitrosophenyl) ether, bis(2-aminophenyl)-4-carbamate) ether, bis(2-nitro-4-aminophenyl) ether, bis(2-nitroso-4-aminophenyl) ether, bis(4-aminophenyl-2-carbamate) ether, $\alpha$-amino-$\alpha'$-nitro-*m*-xylene, $\alpha$-amino-$\alpha'$-nitroso-*m*-xylene, $\alpha$-amino-*m*-xylene-$\alpha'$-carbamate, $\alpha$-amino-$\alpha'$-nitro-*p*-xylene, $\alpha$-amino-$\alpha'$-nitroso-*p*-xylene, $\alpha$-amino-*p*-xylene-$\alpha'$-carbamate, 1-chloro-2-amino-4-nitrobenzene, 1-chloro-2-amino-4-nitrosobenzene, 1-chloro-2-aminobenzene-4-carbamate, 1-chloro-2-nitro-4-amino-benzene, 1-chloro-2-nitroso-4-aminobenzene, 1-chloro-4-aminobenzene-2-carbamate, 1-amino-5-nitronaphthalene, 1-amino-5-nitrosonaphthalene, 1-aminonaphthalene-5-carbamate and the like. The isomers or homologues of these aryl and aralkyl amino compounds are also usable. These aryl and aralkyl amino compounds may be used singly or in combination. It will be noted that the nitro group of aryl or aralkyl nitro compounds are also converted into a corresponding urethane under reaction conditions used in the practice of the invention. Of the above-mentioned amino compounds, nitroaminotoluenes and aminotoluenecarbamates are most preferable because these compounds are more reactive than others.

The hydroxyl group-containing compounds useful in the process of the invention include primary, secondary and tertiary monohydric alcohols and polyhydric alcohols and polyhydric alcohols, and

monohydric phenols and polyhydric phenols. Typical of such compounds are ethanol and phenol. Suitable alcohols may be expressed by a general formula $R(OH)_n$ in which R represents a linear or branched alkyl, a cycloalkyl, an alkylene, a cycloalkylene or an aralkyl group, and *n* is 1 or 2 or a higher integer. These alcohols may further include a substituent containing an oxygen, nitrogen, halogen or sulphur atom such as, for example, halogen, sulphoxide, sulpho, amide, carbonyl or a carboxylic acid ester group.

Examples of the alcohols expressed by the general formula $R(OH)_n$ include monohydric alcohols such as methyl alcohol, ethyl alcohol, *n*- and *iso*-propyl alcohol, *n*-, *iso*- and *t*-butyl alcohol, linear or branched amyl alcohol, hexyl alcohol, cyclohexyl alcohol, lauryl alcohol, cetyl alcohol, benzyl alcohol, chlorobenzyl alcohol, methoxybenzyl alcohol, etc., dihydric alcohols such as ethylene glycol, diethylene glycol, propylene glycol, dipropylene glycol, etc., trihydric alcohols such as glycerol, hexanetriol, etc., and more polyfunctional polyols.

Suitable examples of phenols include phenol itself, chlorophenol, cresol, ethylphenol, linear or branched propylphenol, butyl- and higher alkylphenols, catechol, resorcin, 4,4′-dihydroxy-diphenylmethane, 2,2′-*iso*propylidenediphenyl, anthranol, phenanthrol, pyrogallol, phloroglucinol, etc. Of these, methanol, ethanol and *iso*butanol are preferred since the use of these alcohols results in higher velocity of reaction and higher yield of a final product.

The platinum group metals and/or compounds thereof which are used as an integer of the catalytic system in the process of the invention are palladium, rhodium and ruthenium elements or compounds thereof, or a mixture thereof. Examples of such compounds are halides, cyanides, thiocyanides, *iso*cyanides, oxides, sulphates, nitrates, carbonyl compounds of palladium, rhodium or ruthenium, their addition compounds or complexes with tertiary amines such as triethylamine, pyridine, *iso*quinoline, etc., and their complexes with organic phosphorous compounds such as triphenylphosphine. These catalysts may be used for the reaction as such or may be supported on carriers such as alumina, silica, carbon, barium sulphate, calcium carbonate, asbestos, bentonite, diatomaceous earth, fuller's earth, organic ion-exchange resins, inorganic ion-exchange resins, magnesium silicate, aluminium silicate, molecular sieves and the like. These carriers may be charged into a reactor separately from palladium, rhodium, ruthenium or their compounds. In view of a great catalytic activity, palladium and/or its compounds are preferable to other platinum group metals or compounds thereof.

The Lewis acids and/or compounds thereof are used as the promoter. The term "Lewis acids" herein used is intended to imply those as described, for example, in Physical Organic Chemistry by Jack Hine, 1962 (McGraw Hill Book Co., New York), including Brönsted acids.

Examples of such acids include halides, sulphates, acetates, phosphates, and nitrates such as tin, titanium, germanium, aluminium, iron, nickel, zinc, cobalt, manganese, thallium, zirconium, copper, lead, vanadium, niobium, tantalum, mercury, etc. More particularly, suitable Lewis acids include ferric chloride, ferrous chloride, stannic chloride and copper acetate. Of these, ferrous chloride and ferric chloride are the most preferable. Further, the compounds of the Lewis acids may be, for example, complexes with tertiary amines or organic phosphorous compounds. Examples of such tertiary amines capable of producing complexes include triethylamine, N,N-diethylaniline, N,N-diethylcyclohexylamine, 1,4-diazabicyclo-[2,2,2]octane, and nitrogen-containing heterocyclic compounds wherein nitrogen forms part of the ring system such as pyridine, quinoline and *iso*quinoline. Examples of phosphorous compounds or phosphines include triphenylphosphine, dimethylphenylphosphine, bisdiphenylphosphinoethane and the like. Of these complexes, complexes of ferrous chloride and nitrogen-containing heteroaromatic compounds are preferred since they are less corrosive towards the inside surface of a reactor, can improve the yield of a final product, and permit easier recovery of the catalyst as compared with other promoters.

These complex compounds serve more effectively when used after preparation of the complexes but the starting materials for such complexes may be introduced into the reaction system separately.

If a nitrogen-containing heterocyclic compound is used, aside from the Lewis acid, as the promoter in the reaction system, addition of water in a small amount will facilitate the reaction to proceed at a much higher velocity.

It is desirable that the reaction is conducted using the hydroxyl group-containing organic compound and carbon monoxide in such amounts that the hydroxyl group and carbon monoxide are in at least equimolar or greater ratios to the amino group in the case of an amino compound having a carbamate group and to a total of the amino group and the nitro or nitroso group in the case of an amino compound having a nitro or nitroso group.

The amount of the platinum group metal may vary widely depending on the kind of the amino compound and other reaction conditions but is generally in a range of $1—1 \times 10^{-5}$, preferably $5 \times 10^{-1}—1 \times 10^{-4}$, as metal element, by weight ratio to the amino compound.

The Lewis acid used as the promoter may be used in a range of $2—2 \times 10^{-3}$, preferably $1—5 \times 10^{-2}$, by weight ratio to the amino compound.

The reaction temperature is generally maintained in a range of 80°—230°C, preferably 140—200°C. The reaction pressure is in the range of 10—1000 kg/cm$^2$ gauge, preferably 30—500 kg/cm$^2$ gauge as expressed in terms of the partial pressure of carbon monoxide. Addition of a small amount of water will shorten the reaction time. In this case, the amount of water added is in a range of

1—70 moles per 100 moles, preferably 10—50 moles per 100 moles of the starting primary amine. An amount less than 1 mole of water per 100 moles of the amino compound does not have an appreciable effect on the reaction time, whereas a larger amount lowers the yield of a final product.

The reaction time depends on the property or kind of the amino compound, reaction temperature, reaction pressure, the kind and amount of the catalyst, amount of water and the type of reactor but is generally in a range of 5 minutes to 6 hours.

After the completion of the reaction, the reaction mixture may be cooled. After discharging the gas from the reaction system, the reaction product may be subjected to a treatment by an ordinary separation technique such as filtration, distillation or other suitable means thereby separating the resulting urethane from unreacted materials, by-products, the solvent and catalyst.

The present invention will be illustrated by the following examples, which should not be construed as limiting thereto the present invention. In the examples, all of the reactions were effected in a stainless steel ("SUS 32"), magnetically agitated autoclave. A comparative example is provided between Examples 2 and 3.

## Example 1

10.33 g of 2-amino-4-nitrotoluene, 68 ml of ethanol, 0.0086 g of palladium chloride, 3.71 g of the ferrous chloride-pyridine complex (obtained by interacting one mole of ferrous chloride and two moles pyridine in methanol as solvent), and 0.29 g of water were charged into an autoclave with an inner volume of 200 ml. The air in the autoclave was replaced by nitrogen gas. Then, carbon monoxide was charged into the autoclave under pressure until the initial pressure reached 90 kg/cm$^2$ gauge. The reaction system was heated under agitation and subjected to the reaction at 165°C for 40 min. After completion of the reaction, the system was allowed to cool to room temperature and, after degassing, the reaction solution was analyzed, revealing that the yield of the diurethane, toluene-2,4-diethyl-carbamate was 84.6%. The diurethane produced has the formula

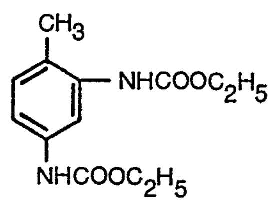

## Example 2

7.8 g of 2-aminotoluene-4-ethylcarbamate 6.0 ml of ethanol, 0.02 g of palladium chloride, and 8.8 g of ferrous chloride-pyridine complex were charged into an autoclave with an inner volume of 200 ml. Carbon monoxide was charged into the reaction system until the initial pressure reached 70 kg/cm$^2$ gauge to undergo the reaction at 160°C for 240 minutes. After completion of the reaction, the reaction solution was analyzed, with the result that the yield of the diurethane, toluene-2,4-diethyl carbamate was 20%.

Comparative Example.

6.3 g of aniline, 68 ml of ethanol, 0.02 g of palladium chloride, and 7.42 g of ferrous chloride-pyridine complex were charged into an autoclave with an inner volume of 200 ml, followed by the urethanation reaction under an initial pressure of carbon monoxide of 90 kg/cm$^2$ gauge at 165°C for 240 minutes. After completion of the reaction, the reaction solution was analyzed, with the result that no N-phenylethylcarbamate was detected.

## Examples 3—8

Various amino compounds other than 2-aminotoluene-4-ethylcarbamate were used to conduct the urethanation reaction in the same manner as in Example 1 using an initial pressure of 90 kg/cm$^2$ gauge, a reaction temperature of 165°C and other reaction parameters indicated in the Table below. The results are also shown in the Table.

TABLE

| Example No. | Amine Compound | Amount (g) | Alcohol | Amount (ml) | Water (g) | $PdCl_2$ (g) | $FeCl_2$-(pyridine)$_2$ (g) | Reaction time (min) | Yield of corresponding diurethane (%) |
|---|---|---|---|---|---|---|---|---|---|
| 3 | *m*-nitroaniline | 9.7 | ethanol | 68 | 0.27 | 0.008 | 2.2 | 40 | 85 |
| 4 | *p*-nitroaniline | 9.7 | ,, | ,, | ,, | ,, | ,, | ,, | 80 |
| 5 | aniline *m*-ethyl-carbamate | 12.5 | ,, | ,, | 0 | 0.032 | 6.3 | 240 | 20 |
| 6 | 4-amino-2-nitrotoluene | 10.7 | ,, | ,, | 0.30 | 0.009 | 2.5 | 40 | 85 |
| 7 | 4-aminotoluene-2-ethyl-carbamate | 13.6 | ,, | ,, | 0 | 0.035 | 6.8 | 240 | 20 |
| 8 | 2-aminotoluene-4-*iso*butyl-carbamate | 15.6 | *iso*butanol | ,, | 0 | 0.040 | 7.8 | 240 | 30 |

**Claims**

1. A process for producing N-aryl or N-aralkyl substituted urethanes by reacting (i) an aryl or aralkyl nitrogen-containing compound, (ii) an organic compound having at least one hydroxyl group, and (iii) carbon monoxide in the presence of a catalyst, characterised in that component (i) is an aryl or aralkyl primary amino compound having a nitro group, a nitroso group or a carbamate group, that the catalyst consists of a catalytic system comprising a metal and/or a compound thereof chosen from palladium, rhodium and ruthenium and compounds thereof and as promoter a Lewis acid and/or a compound thereof, and that the reaction is conducted at elevated temperature and under a carbon monoxide partial pressure of 10—1000 $kg/cm^2$.

2. A process according to Claim 1, wherein component (i) is a nitro amino toluene.

3. A process according to Claim 1, wherein component (i) is an aminotoluene alkylcarbamate.

4. A process according to any preceding claim, wherein the metal and/or a compound thereof is palladium and/or palladium halogenide.

5. A process according to any preceding claim, wherein the promoter is ferrous chloride and/or a compound thereof or ferric chloride and/or a compound thereof.

6. A process according to any one of Claims 1 to 4, wherein the promoter is a complex of ferrous chloride and a nitrogen-containing heterocyclic compound wherein nitrogen forms part of the ring system.

7. A process according to Claim 6, wherein water is added to the reaction system in an amount of 1—70 moles per 100 moles of component (i).

8. A process according to any preceding claim, wherein component (ii) is a compound having the general formula $R(OH)_n$ in which R represents a linear or branched alkyl, a cycloalkyl, an alkylene, a cycloalkylene or an aralkyl group and *n* is 1 or 2 or a higher integer, the compound optionally being substituted by a substituent containing an oxygen, nitrogen, halogen or sulphur atom.

9. A process according to any preceding claim, wherein component (i) is an amino toluene carbamate.

10. A process according to any preceding claim, wherein component (i) is chosen from 2-amino-4-nitrotoluene, 2-aminotoluene-4-ethylcarbamate, *m*-nitroaniline, *p*-nitroaniline, aniline *m*-ethylcarbamate, 4-amino-2-nitrotoluene, 4-aminotoluene-2-ethylcarbamate, and 2-aminotoluene -4-*iso*butylcarbamate.

**Revendications**

1. Procédé de production d'uréthanes substitués en N-aryle ou N-aralcoyle par réaction de (i) un composé contenant un aryl- ou aralkyl-azote, (ii) un composé organique ayant moins un groupe hydroxyle, et (iii) de l'oxyde de carbone en présence d'un catalyseur, caractérisé en ce que le composant (i) est un composé aryl- ou aralkyl-amino primaire ayant un groupe nitro, un groupe nitroso ou un groupe carbamate, en ce que le catalyseur se compose d'un système catalytique comprenant un métal et/ou son composé choisi parmi le palladium, le rhodium et le rhuténium et leurs composés et comme promoteur un acide de Lewis et/ou son composé, et en ce que la réaction est entreprise à une température élevée et sous une pression partielle d'oxyde de carbone de 9,81—981 bars.

2. Procédé selon la revendication 1 où le composant (i) est un nitroaminotoluène.

3. Procédé selon la revendication 1 où le composant (i) est un alkylcarbamate d'aminotoluène.

4. Procédé selon l'une quelconque des revendications précédentes où le métal et/ou son composé est du palladium et/ou un halogénure de palladium.

5. Procédé selon l'une quelconque des revendications précédentes où le promoteur est du chlorure ferreux et/ou son composé ou du chlorure ferrique et/ou son composé.

6. Procédé selon l'une quelconque des revendications 1 à 4, caractérisé en ce que le promoteur est un complexe de chlorure ferreux et d'un composé hétérocyclique contenant de l'azote où l'azote fait partie du système du noyau.

7. Procédé selon la revendication 6 où de l'eau est ajoutée au système réactionnel en une quantité de 1—70 moles pour 100 moles du composant (i).

8. Procédé selon l'une quelconque des revendications précédentes où le composant (ii) est un composé ayant pour formule générale $R(OH)_n$ où R représente un groupe alcoyle linéaire ou ramifié, cycloalcoyle, alcoylène ou cycloalcoylène ou aralcoyle et *n* est 1 ou 2 ou un nombre entier supérieur, le composé étant éventuellement substitué par un substituant contenant un atome d'oxygène, d'azote, d'halogène ou de soufre.

9. Procédé selon l'une quelconque des revendications précédentes, où le composant (i) est un carbamate d'aminotoluène.

10. Procédé selon l'une quelconque des revendications précédentes où le composant (i) est choisi parmi le 2-amino-4-nitrotoluène, le 2-aminotoluène-4-éthylcarbamate, la m-nitroaniline, la p-nitroaniline, le m-éthylcarbamate d'aniline, le 4-amino-2-nitrotoluène, le 4-aminotoluène-2-éthylcarbamate et le 2-aminotoluène-4-isobutylcarbamate.

**Patentansprüche**

1. Verfahren zur Herstellung N-Aryl- oder N-Aralkyl-substituierter Urethane durch Umsetzen (i) einer Aryl- oder Aralkylstickstoff-haltigen Verbindung, (ii) einer organischen Verbindung mit wenigstens einer Hydroxyl-Gruppe und (iii) Kohlenmonoxid in Gegenwart eines Katalysators, dadurch gekennzeichnet, daß die Komponente (i) eine Aryl- oder Aralkyl-prim.-Amino-Verbindung mit einer Nitro-Gruppe, einer Nitroso-Gruppe oder einer Carbamat-Gruppe ist, daß der Katalysator aus einem katalytischen System mit einem Metall und/oder einer Verbindung von diesem, ausgewählt unter Palladium, Rhodium und Ruthenium und deren Verbindungen, und als Promoter einer Lewis-Säure und/oder einer Verbindung hiervon besteht, und daß die Umsetzung bei erhöhter Temperatur und unter einem Kohlenmonoxid-Partialdruck von 10—1000 kg/cm$^2$ erfolgt.

2. Verfahren nach Anspruch 1, bei dem die Komponente (i) ein Nitroaminotoluol ist.

3. Verfahren nach Anspruch 1, bei dem die Komponente (i) ein Aminotoluol-alkylcarbamat ist.

4. Verfahren nach einem vorhergehenden Anspruch, bei dem das Metall und/oder eine Verbindung hiervon Palladium und/oder Palladiumhalogenid ist.

5. Verfahren nach einem vorhergehenden Anspruch, bei dem der Promoter Eisen(II)chlorid und/oder eine Verbindung hiervon oder Eisen(III)chlorid und/oder eine Verbindung hiervon ist.

6. Verfahren nach einem der Ansprüche 1 bis 4, bei dem der Promoter ein Komplex aus Eisen(II)chlorid und einer stickstoffhaltigen heterocyclischen Verbindung ist, wobei der Stickstoff einen Teil des Ringsystems bildet.

7. Verfahren nach Anspruch 6, bei dem Wasser dem Reaktionssystem in einer Menge von 1—70 Mol pro 100 Mol der Komponente (i) zugesetzt wird.

8. Verfahren nach einem vorhergehenden Anspruch, bei dem die Komponente (ii) eine Verbindung der allgemeinen Formel $R(OH)_n$ ist, wobei R eine lineare oder verzweigte Alkyl-, eine Cycloalkyl-, eine Alkylen-, eine Cycloalkylen- oder eine Aralkyl-Gruppe bedeutet und n 1 oder 2 oder eine höhere ganze Zahl ist, wobei die Verbindung gegebenenfalls mit einem ein Sauerstoff-, Stickstoff-, Halogen- oder Schwefelatom enthaltenden Substituenten substituiert ist.

9. Verfahren nach einem vorhergehenden Anspruch, bei dem die Komponente (i) ein Aminotoluolcarbamat ist.

10. Verfahren nach einem vorhergehenden Anspruch, bei dem die Komponente (i) unter 2-Amino-4-nitrotoluol, 2-Aminotoluol-4-äthylcarbamat, m-Nitroanilin, p-Nitroanilin, Anilin-m-äthylcarbamat, 4-Amino-2-nitrotoluol, 4-Aminotoluol-2-äthylcarbamat und 2-Aminotoluol-4-isobutylcarbamat ausgewählt ist.